# FASCICULE DE BREVET EUROPEEN

(11) **EP 3 544 494 B1**
(45) Date de publication et mention de la délivrance du brevet: **28.05.2025**
(21) Numéro de dépôt: 17822725.2
(22) Date de dépôt: 28.11.2017
(51) Int. Cl.: A61B 5/026, A61B 5/0295, A61B 5/00

(54) **APPAREILLAGE DE MONITORAGE DE PERFUSION D'UNE MUQUEUSE RECTALE**
AUSRÜSTUNG ZUR ÜBERWACHUNG DER PERFUSION EINER REKTALE MUKOSA
EQUIPMENT FOR MONITORING PERFUSION OF A RECTAL MUCOSA

(30) Priorité: 28.11.2016 FR 1661555
(43) Date de publication de la demande: 02.10.2019
(73) Titulaire: Vygon, 95440 Ecouen (FR)
(72) Inventeur: ALLAOUCHICHE, Bernard, 69008 Lyon (FR); DELCROIX, Benjamin, 06270 Villeneuve-Loubet (FR); FALCON, Dominique, 69003 Lyon (FR); BONIDAL, Rémi, 69100 Villeurbanne (FR)
(74) Mandataire: Rhein, Alain
(86) Numéro de dépôt international: PCT/FR2017/053279
(87) Numéro de publication internationale: WO 2018/096303

(56) Documents cités:
- WO-A1-2013/127819
- WO-A1-2013/149264
- WO-A1-2015/190994
- US-A1- 2002 173 731

## Description

La présente invention concerne le domaine des appareillages de mesure pour estimer les paramètres métaboliques relatif à la perfusion d'un organe d'un patient, en particulier un organe comprenant une muqueuse et des tissus musculaires.

Un tel appareillage peut être utilisé dans de nombreuses applications pour surveiller les patients susceptibles d'hypo-perfusion tissulaire. Ces affections peuvent être d'origines diverses : hémorragiques, infectieuses ou inflammatoires.

Un suivi des paramètres biologiques relatifs à la perfusion fournit notamment à l'utilisateur des informations relatives à la microcirculation de l'organe pour l'aider dans le choix des traitements adaptés au patient. En effet, la connaissance de l'état de la perfusion locale, en particulier de la muqueuse digestive, est un élément important pour permettre des prises en charge des patients pouvant présenter des altérations de cette perfusion.

Par exemple, la technologie de la photo-pléthysmographie est un moyen permettant d'évaluer la perfusion d'un tissu sur une profondeur connue. Malheureusement concernant en particulier des tissus tels que ceux du rectum, les mesures peuvent être perturbées par l'écoulement de matières fécales à l'interface entre le capteur et la paroi. Si cet aspect est contrecarré par un bouchon, des contractions involontaires du rectum pour expulser le bouchon vont entrainer une modification du flux sanguin dans la zone de mesure et perturber les mesures.

On connait par le document WO 2013/149264 un appareillage permettant de mesurer une perfusion de la paroi de l'œsophage. L'appareillage est également applicable à l'intégralité du tractus gastro-intestinal. Il comprend une sonde équipée d'un système d'accroche par succion à la paroi gastrointestinale, d'un Doppler laser pour mesurer la circulation sanguine, d'une sonde ultrason pour capturer l'activité musculaire.

On connait également par le document US 2002/0173731 un appareillage de monitorage de la muqueuse du rectum qui comprend des moyens d'application de chaleur à la paroi de la muqueuse et un capteur thermique mesurant la réponse de la muqueuse en fonction de la perfusion du tissu. L'appareillage comprend en outre des moyens d'estimation du choc en fonction de la réponse thermique de la muqueuse du rectum.

Un objectif de l'invention est de proposer un appareillage de monitorage de la perfusion d'un tissu dont les mesures sont moins perturbées que dans l'art antérieur.

Pour atteindre cet objectif l'invention propose un appareillage de monitorage de perfusion d'une muqueuse rectale tel que défini dans les revendications.

Avantageusement, l'appareillage selon l'invention permet d'associer des données d'un capteur de perfusion à celles d'un capteur d'activité musculaire afin d'obtenir un monitorage de la perfusion tenant compte de l'activité musculaire, en particulier des périodes de contractions du rectum pour expulser un bouchon en amont du dispositif.

Plus particulièrement, l'unité de traitement reçoit en entrée des données de mesure de perfusion et des données d'activité musculaire, et permet de tenir compte des données d'activité musculaire pour le monitorage de la perfusion du tissu. De préférence, les données de mesure sont invalidées pendant les périodes d'activité musculaire.

L'invention sera davantage détaillée par la description de modes de réalisation non limitatifs, et sur la base des figures annexées, dans lesquelles :
- la figure 1 est un schéma d'un appareillage selon l'invention, inséré dans un rectum ;
- la figure 2 est un appareillage selon un premier mode de réalisation de l'invention ; et
- la figure 3 est un appareillage selon un deuxième mode de réalisation de l'invention.

La présente invention concerne appareillage de monitorage de perfusion d'une muqueuse rectale et comprend un dispositif de monitorage 1 de perfusion d'une muqueuse rectale. En particulier, le dispositif 1 comprend une sonde sous forme de cathéter. De préférence, le corps 1a du cathéter n'est pas rigide.

Un tel dispositif 1 comprend un capteur de perfusion 2 configuré pour détecter une perfusion du tissu. Le capteur de perfusion 2 est un capteur utilisant la technologie de la photo-pléthysmographie, à savoir un capteur photopléthysmographique. Ce type de capteur 2 associé à un tel dispositif 1 et la mesure de perfusion associée ont été présentés en détail notamment dans les demandes WO2013127819 et WO2015044336, concernant des mesures dans un tissu particulier, à savoir une muqueuse duodénale.

Pour qu'un capteur de photo-pléthysmographie puisse donner une indication de perfusion fiable dans le temps, il faut que le chemin des photons entre l'émetteur et le récepteur soit constant dans une application donnée. En particulier, il importe premièrement que le capteur soit plaqué contre la paroi, deuxièmement que l'interface entre le capteur et la paroi de la muqueuse soit toujours la même, et troisièmement, que la composition du tissu reste inchangée.

Dans le cas d'une mesure rectale comme pour une mesure duodénale, le premier point est résolu par une approche du même type que pour une sonde duodénale décrite dans les demandes mentionnées ci-dessus, en particulier un ballon ou un stent permettant de plaquer le capteur contre la paroi.

Concernant le deuxième point, dans le cadre d'une mesure dans un autre tissu particulier tel qu'une muqueuse de rectum 3, des selles peuvent venir se placer entre le capteur de perfusion 2 et la paroi et perturber les mesures.

Concernant le troisième point, il ne pose pas de problème particulier sauf dans le cas où les muscles entourant l'ampoule rectale se contractent afin d'expulser les selles. Dans ce cas, la muqueuse est écrasée entre les muscles et le contenu de l'ampoule rectale ce qui résulte à une densification du réseau sanguin sans augmenter le flux total. Cette densification peut perturber les mesures de perfusion.

Une première solution est de bloquer l'arrivée des selles en amont du point de mesure afin d'éliminer la corruption de la mesure en raison en raison de selles s'intercalant entre le capteur 2 et la muqueuse. Par exemple, un bouchon peut être utilisé.

Malheureusement, cette solution est problématique au sens où les selles vont s'accumuler au-dessus du bouchon. Au-delà d'une certaine quantité de selles, la pression des selles sur la muqueuse va dépasser un seuil, estimé à 3 kPa, ce qui va déclencher le réflexe d'expulsion.

Ce réflexe d'expulsion va modifier la densité du réseau capillaire entrainant une valeur de l'indicateur de perfusion calculé erroné en ce sens qu'il ne sera plus comparable aux valeurs précédentes.

Il est particulièrement important de connaitre le début de l'activité musculaire afin d'en tenir compte pour les mesures de perfusion. C'est d'autant plus important que l'activité musculaire peut commencer bien avant l'expulsion réelle.

Pour pallier à ces inconvénients, le dispositif selon l'invention comprend en outre au moins un capteur d'activité 4 configuré pour détecter une activité musculaire de l'organe, ledit au moins un capteur d'activité étant disposé à proximité du capteur de perfusion 2. Le capteur de perfusion 2 et le capteur d'activité 4 sont configurés pour être associés à une unité de traitement 5.

Plus particulièrement, l'unité de traitement 5 reçoit en entrée des données de mesure de perfusion et des données d'activité musculaire, et permet de tenir compte des données d'activité musculaire pour le monitorage de la perfusion du tissu. De préférence, les données de mesure de perfusion sont invalidées pendant les périodes d'activité musculaire.

Selon l'invention, le dispositif de monitorage 1 comprend une section d'occlusion 6 comprenant le capteur d'activité 4, et une section de mesure de perfusion 7 comprenant le capteur de perfusion 4. En particulier, la section d'occlusion 6 est disposée en amont de la section de mesure de perfusion 7 en référence au sens du transit.

Avantageusement, la section d'occlusion 6 est une partie du dispositif, et permet de s'abstenir d'un bouchon qui serait difficile à retirer après utilisation.

En particulier, la section d'occlusion 6 est configurée pour réaliser une « semi-occlusion » c'est-à-dire qu'elle bloque partiellement soit les selles solides et les selles liquide, soit uniquement les selles solides ; et elle est expulsable par les muscles du rectum 3.

Avantageusement, la section d'occlusion 6 permet d'avoir un espace de mesure autour de la section de mesure 7 sans perturbation des mesures par des selles venant s'intercaler entre le capteur et la muqueuse du rectum 3.

Selon l'invention, la section d'occlusion 6 est mobile entre une position repliée dans laquelle elle a une petite taille dans un sens radial et une position déployée dans laquelle elle a une grande taille dans le sens radial.

Avantageusement, la section d'occlusion 6 peut prendre une première configuration permettant une insertion facilitée, à savoir la position repliée, et une deuxième configuration permettant une bonne semi-occlusion du rectum, à savoir la position déployée.

La section d'occlusion 6 peut être gonflable. Par exemple elle comprend un ballonnet. Avantageusement, une section d'occlusion gonflable permet un changement de configuration simplifié par simple gonflage/dégonflage de la section d'occlusion.

La section d'occlusion 6 peut comprendre un stent. Avantageusement, un stent permet d'avoir une position déployée rigide.

La section d'occlusion 6 peut comprendre un matériau à mémoire de forme tel qu'un plastique à mémoire de forme.

Selon l'invention, la section de mesure de perfusion 7, est de préférence gonflable, et est mobile entre une position repliée dans laquelle elle a une petite taille dans un sens radial et une position déployée dans laquelle elle a une grande taille dans le sens radial.

Les avantages de la section d'occlusion 6 déployable sont applicables mutatis mutandis à la section de perfusion 7 déployable.

Selon une variante, le capteur d'activité 4 est un capteur d'effort 4a disposé sur la section d'occlusion 6. En particulier, le capteur d'effort 4a est disposé entre la section d'occlusion 6 et la muqueuse du rectum 3. Le capteur d'effort 4a mesure la pression mécanique de la muqueuse sur la section d'occlusion 6. Avantageusement, un capteur d'effort 4a permet de mesurer localement l'activité musculaire de la muqueuse.

Selon une variante, le capteur d'activité 4 est un capteur de pression locale 4b. En particulier, le capteur de pression locale 4b est disposé dans la section d'occlusion 6. Le capteur de pression locale 4b mesure la pression pneumatique dans la section d'occlusion 6. Avantageusement, un capteur de pression locale 4b permet de mesurer localement l'activité musculaire de la muqueuse par l'intermédiaire de la mesure de la pression pneumatique.

Selon une variante, la section de mesure de perfusion 7 comprend un deuxième capteur d'activité 4 pouvant être l'un des capteurs d'activité décrits ci-dessus. De préférence, le deuxième capteur d'activité 4 est un dispositif de mesure de pression pneumatique 4c externe. En particulier, ce dispositif 4c est disposé sur une section non introduite dans le rectum 3.

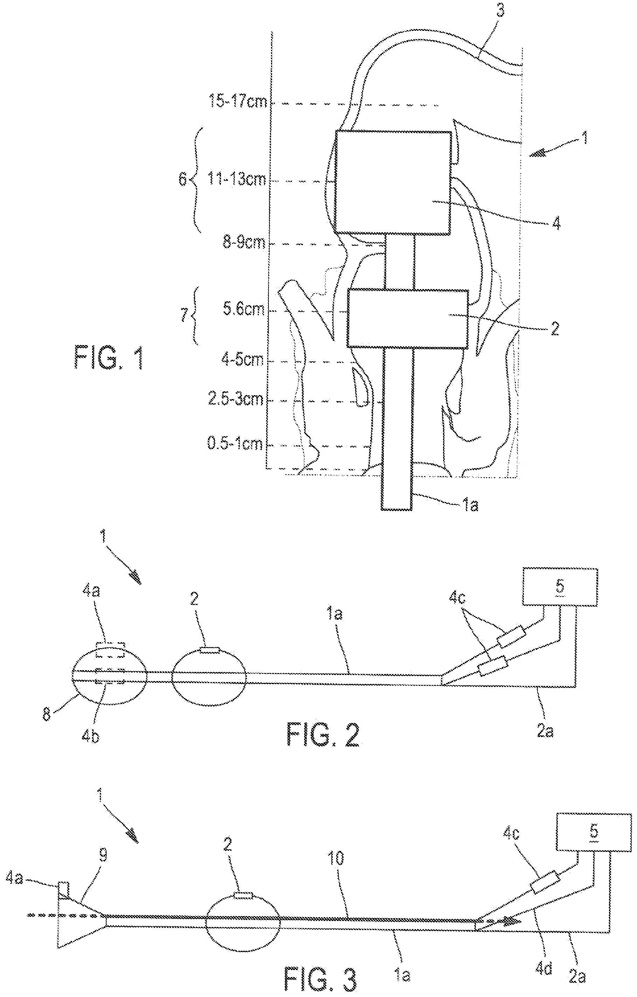

Selon un premier mode de réalisation, la section d'occlusion 6 comprend un ballonnet 8 pouvant être ovoïde. Cette variante est en particulier illustrée dans la figure 2.

Le ballonnet 8 peut comprendre un capteur d'effort 4a sur le pourtour. Alternativement ou en combinaison, le ballonnet peut comprendre un capteur de pression 4b à l'intérieur.

De préférence, le ballonnet 8 est associé à un dispositif de mesure de pression 4c externe pour mesurer la pression pneumatique du ballonnet 8.

La variante préférée du premier mode de réalisation comprend :
- un ballonnet bloquant 8 d'un diamètre supérieur à 2 cm et inférieur à 6cm correspondant à une dilatation maximale de l'ampoule rectale ;
- un ballonnet de mesure sur la section de mesure 7, d'une taille d'environ 3cm configuré pour être disposé à proximité du ballonnet bloquant 8, par exemple à environ 2cm. Ce ballonnet de mesure est équipé d'un capteur de photo-pléthysmographie. En particulier, le capteur est à l'intérieur du ballon mais fait face à la paroi.
- un corps de cathéter 1a de préférence creux ; et
- une unité d'acquisition et traitement qui analyse les variations de pressions pour signaler l'activité rectale.

Le corps 1a creux du cathéter permet de laisser passer :
- des câbles 2a du capteur de perfusion 2 ;
- un canal d'arrivée d'air pour gonfler le ballonnet de mesure (7). Cette même arrivée d'air est connectée à un capteur de pression 4c ; et
- un deuxième canal d'arrivée d'air pour gonfler le ballonnet 8 semi-occlusif. Cette même arrivée d'air est connectée à un capteur de pression 4c.

Selon un deuxième mode de réalisation, la section d'occlusion 6 comprend un réceptacle 9 et un canal d'évacuation 10. Cette variante est en particulier illustrée dans la figure 3. Ainsi, selon un deuxième mode de réalisation, la section d'occlusion a une forme de réceptacle telle qu'une forme de cône se prolongeant par ledit canal d'évacuation 10. Avantageusement, le canal d'évacuation permet l'évacuation des selles liquides et peut ainsi limiter la pression des selles sur la section d'occlusion 6.

Le réceptacle 9 peut être mis en position déployée par l'intermédiaire d'un stent, par un dispositif gonflable tel qu'un ballonnet ou par un dispositif à mémoire de forme tel qu'un plastique à mémoire de forme.

Le réceptacle 9 comprend de préférence un capteur d'effort 4a à sa surface, en particulier sur le stent ou sur le dispositif à mémoire de forme. Alternativement ou en combinaison, le réceptacle peut comprendre un capteur de pression à l'intérieur du ballonnet le cas échéant. Dans ce cas, le ballonnet peut être un ballonnet torique.

La variante préférée du deuxième mode de réalisation comprend :
- un entonnoir 9 à mémoire de forme à plusieurs lèvres, d'un diamètre maximal supérieur à 3cm et inférieur à 4cm. La partie de plus grand diamètre est équipée d'un capteur d'effort 4a pour une mesure de contrainte axiale.
- un ballonnet de mesure sur la section de mesure 7, d'une taille d'environ 3cm configuré pour être disposé à proximité, par exemple à 2cm de la partie la plus petite de l'entonnoir 9. Ce ballonnet de mesure (7) est équipé d'un capteur de photo-pléthysmographie 2. En particulier, le capteur est à l'intérieur du ballon mais fait face à la paroi.
- un corps de cathéter 1a de préférence creux ; et
- une unité d'acquisition et traitement 5 qui analyse les variations de pressions pour signaler l'activité rectale.

Le corps 1a du cathéter creux permet de laisser passer :
- des câbles 2a du capteur de photo-pléthysmographie ;
- des câbles 4d du capteur d'effort ;
- un canal d'arrivée d'air pour gonfler le ballonnet de mesure. Cette même arrivée d'air est connectée à un capteur de pression 4c.

L'invention porte sur un appareillage de monitorage de perfusion d'une muqueuse rectale, comprenant un dispositif 1 tel que décrit précédemment et une unité de traitement 5 associée auxdits capteurs 2, 4.

Le suivi des données de mesures se fait dans le temps. Les détails d'expérimentation tels que les unités de mesure de perfusion ou d'effort, et la calibration des instruments sont connus en eux même de l'homme du métier ou sont exposées dans les demandes internationales citées ci-dessus.

## Revendications

1. Appareillage (1) de monitorage de perfusion d'une muqueuse rectale comprenant un dispositif de monitorage de perfusion d'une muqueuse rectale, comprenant :
- une section de mesure (7) de perfusion qui est mobile entre une position repliée dans laquelle elle a une petite taille dans un sens radial et une position déployée dans laquelle elle a une grande taille dans le sens radial,
- un capteur de perfusion (2) de type photopléthysmographique configuré pour détecter une perfusion de la muqueuse rectale, le capteur de perfusion (2) étant monté sur la section de mesure (7) de façon à être plaqué contre la muqueuse rectale lorsque la section de mesure (7) est déployée dans un rectum,
- une section d'occlusion (6) mobile entre une position repliée dans laquelle elle a une petite taille dans un sens radial et une position déployée dans laquelle elle a une grande taille dans le sens radial pour réaliser une semi-occlusion du rectum,
- au moins un capteur d'activité (4) configuré pour détecter une activité musculaire de la muqueuse rectale, ledit au moins un capteur d'activité (4) étant disposé, sur ou dans la section d'occlusion (6) à proximité du capteur de perfusion (2), l'appareillage de monitorage comprenant par ailleurs une unité de traitement (5) configurée pour traiter les données du capteur de perfusion (2) en tenant compte des données du capteur d'activité (4).

2. Appareillage de monitorage (1) selon la revendication 1, dans lequel la section d'occlusion (6) comprend un ballonnet, un stent ou un matériau à mémoire de forme, configuré pour faire passer la section d'occlusion (6) entre 1a position repliée et la position déployée.

3. Appareillage de monitorage (1) selon l'une des revendications 1 et 2, dans lequel la section de mesure de perfusion (7) est gonflable.

4. Appareillage de monitorage (1) selon l'une des revendications 1 à 3, dans lequel le capteur d'activité (4) est un capteur de pression, un capteur d'effort et/ou un dispositif de mesure de pression pneumatique.

5. Appareillage de monitorage (1) selon l'une des revendications 1 à 4, dans lequel la section de mesure de perfusion (7) comprend un deuxième capteur d'activité musculaire (4c).

6. Appareillage de monitorage (1) selon l'une des revendications 1 à 5, dans lequel la section d'occlusion (6) comprend un réceptacle (9) et un canal d'évacuation (10).

7. Appareillage de monitorage (1) selon l'une des revendications 1 à 6, dans lequel la section d'occlusion (6) est disposée en amont de la section de mesure de perfusion (7) en référence au sens du transit.

## Patentansprüche

1. Einrichtung (1) zur Überwachung der Perfusion einer Rektalschleimhaut, umfassend eine Vorrichtung zur Überwachung der Perfusion einer Rektalschleimhaut, die Folgendes umfasst:
- einen Perfusionsmessabschnitt (7), der zwischen einer zusammengeklappten Position, in der er in radialer Richtung eine kleine Größe aufweist, und einer entfalteten Position, in der er radialer Richtung eine große Größe in aufweist, beweglich ist,
- einen Perfusionssensor (2) vom photoplethysmographischen Typ, der zum Erfassen einer Perfusion der Rektalschleimhaut konfiguriert ist, wobei der Perfusionssensor (2) so auf dem Messabschnitt (7) montiert ist, dass er gegen die Rektalschleimhaut gedrückt wird, wenn der Messabschnitt (7) in einem Rektum entfaltet wird,
- einen Okklusionsabschnitt (6), der zwischen einer zusammengeklappten Position, in der er in radialer Richtung eine kleine Größe aufweist, und einer entfalteten Position, in der er in radialer Richtung eine große Größe aufweist, beweglich ist, um eine Teilokklusion des Rektums zu erreichen,
- mindestens einen Aktivitätssensor (4), der dazu konfiguriert ist, die Muskelaktivität der Rektalschleimhaut zu erfassen, wobei der mindestens eine Aktivitätssensor (4) am oder im Okklusionsabschnitt (6) in der Nähe des Perfusionssensors (2) angeordnet ist, wobei die Überwachungseinrichtung ferner eine Verarbeitungseinheit (5) umfasst, die dazu konfiguriert ist, die Daten des Perfusionssensors (2) unter Berücksichtigung der Daten des Aktivitätssensors (4) zu verarbeiten.

2. Überwachungseinrichtung (1) nach Anspruch 1, wobei der Okklusionsabschnitt (6) einen Ballon, einen Stent oder ein Formgedächtnismaterial umfasst, der/das dazu konfiguriert ist, den Okklusionsabschnitt (6) zwischen der zusammengeklappten Position und der entfalteten Position zu überführen.

3. Überwachungseinrichtung (1) nach einem der Ansprüche 1 und 2, wobei der Perfusionsmessabschnitt (7) aufblasbar ist.

4. Überwachungseinrichtung (1) nach einem der Ansprüche 1 bis 3, wobei der Aktivitätssensor (4) ein Drucksensor, ein Kraftsensor und/oder ein pneumatisches Druckmessgerät ist.

5. Überwachungseinrichtung (1) nach einem der Ansprüche 1 bis 4, wobei der Perfusionsmessabschnitt (7) einen zweiten Muskelaktivitätssensor (4c) umfasst.

6. Überwachungseinrichtung (1) nach einem der Ansprüche 1 bis 5, wobei der Okklusionsabschnitt (6) einen Aufnahmebehälter (9) und einen Auslasskanal (10) umfasst.

7. Überwachungseinrichtung (1) nach einem der Ansprüche 1 bis 6, wobei der Okklusionsabschnitt (6) in Bezug auf die Richtung der Passage vor dem Perfusionsmessabschnitt (7) angeordnet ist.

## Claims

1. Apparatus (1) for monitoring perfusion of a rectal mucosa comprising a device for monitoring perfusion of a rectal mucosa, comprising:
- a perfusion measuring portion (7) which is movable between a folded position in which it has a small size in a radial direction and a deployed position in which it has a large size in the radial direction,
- a photoplethysmographic perfusion sensor (2) designed to detect a perfusion of the rectal mucosa, the perfusion sensor (2) being mounted on the measuring portion (7) so as to be pressed against the rectal mucosa when the measuring portion (7) is deployed in a rectum,
- an occlusion portion (6) movable between a folded position in which it has a small size in a radial direction and a deployed position in which it has a large size in the radial direction in order to perform a semi-occlusion of the rectum,
- at least one activity sensor (4) designed to detect a muscular activity of the rectal mucosa, said at least one activity sensor (4) being arranged on or in the occlusion portion (6) in the vicinity of the perfusion sensor (2), the monitoring apparatus further comprising a processing unit (5) designed to process the data from the perfusion sensor (2) taking into account the data from the activity sensor (4).

2. Monitoring apparatus (1) according to claim 1, wherein the occlusion portion (6) comprises a balloon, a stent or a shape-memory material, designed to move the occlusion portion (6) between the folded position and the deployed position.

3. Monitoring apparatus (1) according to either claim 1 or claim 2, wherein the perfusion measuring portion (7) is inflatable.

4. Monitoring apparatus (1) according to any of claims 1 to 3, wherein the activity sensor (4) is a pressure sensor, a stress sensor and/or a pneumatic pressure measuring device.

5. Monitoring apparatus (1) according to any of claims 1 to 4, wherein the perfusion measurement portion (7) comprises a second muscle activity sensor (4c).

6. Monitoring apparatus (1) according to any of claims 1 to 5, wherein the occlusion portion (6) comprises a receptacle (9) and an evacuation channel (10).

7. Monitoring apparatus (1) according to any of claims 1 to 6, wherein the occlusion portion (6) is arranged upstream of the perfusion measurement portion (7) referring to the transit direction.
